(19) Europäisches Patentamt
European Patent Office
Office européen des brevets

(11) Veröffentlichungsnummer: **0 281 891**
**A2**

(12) # EUROPÄISCHE PATENTANMELDUNG

(21) Anmeldenummer: **88103027.4**

(22) Anmeldetag: **01.03.88**

(51) Int. Cl.⁴: **C07C 121/78**, C07C 101/62, C07C 97/10, C07D 295/12, C07D 215/12, C07D 241/42, B41M 5/12, B41M 5/26, B41M 5/20

(30) Priorität: **10.03.87 DE 3707548**

(43) Veröffentlichungstag der Anmeldung:
**14.09.88 Patentblatt 88/37**

(84) Benannte Vertragsstaaten:
**CH DE FR GB IT LI**

(71) Anmelder: **BAYER AG**
**Konzernverwaltung RP Patentabteilung**
**D-5090 Leverkusen 1 Bayerwerk(DE)**

(72) Erfinder: **Eckstein, Udo, Dr.**
**Wolfskaul 8**
**D-5000 Köln 80(DE)**
Erfinder: **Psaar, Hubertus, Dr.**
**Paul-Klee-Strasse 21**
**D-5090 Leverkusen(DE)**
Erfinder: **Raue, Roderich, Dr.**
**Berta-von-Suttner-Strasse 48**
**D-5090 Leverkusen(DE)**

(54) **Farbbildner, ihre Herstellung und Verwendung.**

(57) Farbbildner der allgemeinen Formel

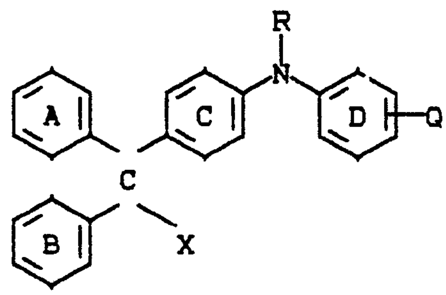

(I),

worin

X Hydroxy, Alkoxy, Alkenyloxy, Aralkoxy, Cycloalkoxy, Aryloxy, Acyloxy, Alkoxycarbonyloxy, Alkylamino, Dialkylamino, Acylamino, Aralkylamino oder Arylamino,

Q Cyano, Alkoxycarbonyl, Aryloxycarbonyl, Aralkoxycarbonyl, Carbamoyl, N-Alkylcarbamoyl, N,N-Dialkylcarbamoyl, N-Alkyl-N-aryl-carbamoyl, Acyl, Alkoxysulfonyl, Aralkoxysulfonyl, Sulfamoyl, N-Alkylsulfamoyl, N,N-Dialkylsulfamoyl, Alkylsulfonyl, Arylsulfonyl oder Aralkylsulfonyl und

R Alkyl, Alkenyl oder Aralkyl bedeuten,

den Ringen A, B, C und D weitere isocyclische oder heterocyclische Ringe anelliert werden können, und die cyclischen und acyclischen Reste sowie die Ringe A, B, C und D weitere Substituenten tragen können, werden zur Herstellung von druckempfindlichen Aufzeichnungsmaterialien verwendet.

EP 0 281 891 A2

## Farbbildner, ihre Herstellung und Verwendung

Gegenstand der Erfindung sind Farbbildner der allgemeinen Formel

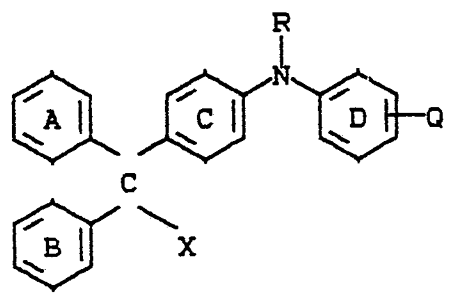

(I),

worin

X Hydroxy, Alkoxy, Alkenyloxy, Aralkoxy, Cycloalkoxy, Aryloxy, Acyloxy, Alkoxycarbonyloxy, Alkylamino, Dialkylamino, Acylamino, Aralkylamino oder Arylamino,

Q Cyano, Alkoxycarbonyl, Aryloxycarbonyl, Aralkoxycarbonyl, Carbamoyl, N-Alkylcarbamoyl, N,N-Dialkylcarbamoyl, N-Alkyl-N-aryl-carbamoyl, Acyl, Alkoxy sulfonyl, Aralkoxysulfonyl, Sulfamoyl, N-Alkylsulfamoyl, N,N-Dialkylsulfamoyl, Alkylsulfonyl, Arylsulfonyl oder Aralkylsulfonyl und

R Alkyl, Alkenyl oder Aralkyl bedeuten,

den Ringen A, B, C und D weitere isocylische oder heterocyclische Ringe anelliert werden können, und die cyclischen und acyclischen Reste sowie die Ringe A, B, C und D weitere in der Farbstoffchemie übliche, nichtionische Substituenten tragen können, oder deren Gemische, ihre Herstellung und ihre Verwendung zur Herstellung von druckempfindlichen Aufzeichnungsmaterialien.

In der Farbchemie übliche nichtionische Substituenten sind z.B. Halogen, Hydroxy, Alkoxy, Alkenyloxy, Aryloxy, Aralkoxy, Cycloalkyloxy, Heteryloxy, Aryl, Heteryl, Alkylmercapto, Arylmercapto, Aralkylmercapto, Alkylsulfonyl, Cyan, Carbamoyl, Alkoxycarbonyl, Amino, das durch 1 oder 2 Alkyl-, Cycloalkyl-, Aryl-oder Aralkylgruppen substituiert sein kann, bevorzugt zu einem 5-oder 6-gliedrigen Ring, oder dessen Substituenten ringgeschlossen sein können. Acylamino, Alkenyloxy, Alkylcarbonyloxy und Arylcarbonyloxy und als Substituenten der Ringe außerdem Alkyl, Aryl, Aralkyl, Alkenyl oder Arylvinyl.

Alkyl steht für $C_1$-$C_{30}$-Alkyl, insbesondere für $C_1$-$C_{12}$-Alkyl.

Die Alkylreste und die Alkylreste in Alkoxy-, Alkylthio-, Alkylamino-, Alkanoylamino-, Alkylsulfonyl-und Alkoxycarbonylgruppen können verzweigt und beispielsweise durch Fluor, Chlor, $C_1$-bis $C_4$-Alkoxy, Cyano oder $C_1$-$C_4$-Alkoxycarbonyl substituiert sein.

Aralkyl ist insbesondere Phenyl-$C_1$-bis -$C_4$-alkyl, das im Phenylkern durch Halogen, $C_1$-bis $C_4$-Alkyl und/oder $C_1$-bis $C_4$-Alkoxy substituiert sein kann.

Cycloalkyl ist insbesondere gegebenenfalls durch Methyl substituiertes Cyclopentyl oder Cyclohexyl.

Alkenyl ist insbesondere $C_2$-$C_5$-Alkenyl, das durch Hydroxy,$C_1$-bis $C_4$-Alkoxy, Cyan, $C_1$-bis $C_4$-Alkoxycarbonyl, Chlor oder Brom monosubstituiert sein kann. Bevorzugt sind Vinyl und Allyl.

Halogen ist insbesondere Fluor, Chlor und Brom, vorzugsweise Chlor.

Aryl ist insbesondere, gegebenenfalls durch ein bis drei $C_1$-bis $C_4$-Alkyl, Chlor, Brom, Cyan, $C_1$-bis $C_4$-Alkoxycarbonyl oder $C_1$-bis $C_4$-Alkoxy substituiertes Phenyl oder Naphthyl.

Alkoxy ist insbesondere gegebenenfalls durch Chlor oder $C_1$-$C_4$-Alkoxy substituiertes $C_1$-$C_{12}$-Alkoxy.

Acyl ist insbesondere $C_1$-bis $C_4$-Alkylcarbonyl oder Benzoyl.

Alkoxycarbonyl ist insbesondere gegebenenfalls durch Hydroxy, Halogen oder Cyan substituiertes $C_1$-bis $C_4$-Alkoxycarbonyl.

Heteryl ist insbesondere Pyridyl, Pyrimidyl, Pyrazinyl, Triazinyl, Indolyl, Imidazolyl, Oxazolyl, Thiazolyl, Triazolyl, Thiadiazolyl oder Tetrazolyl, die benzanelliert sein können, sowie ihre teilhydrierten oder ganz hydrierten Derivate.

Bevorzugte nichtionische Substituenten der Ringe sind $C_1$-$C_4$-Alkyl, $C_1$-$C_4$-Alkoxy, Cyan und Halogen.

Von den Farbbildnern der Formel I haben die Verbindungen der Formel

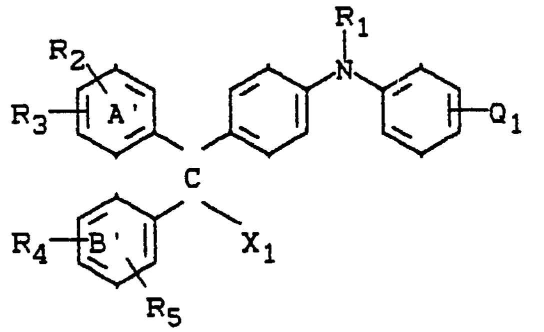

(II)

besondere Bedeutung, worin

$X_1$ Hydroxy; gegebenenfalls durch Chlor oder $C_1$-$C_4$-Alkoxy substituiertes $C_1$-$C_{12}$-Alkoxy; $C_2$-$C_{12}$-Alkenyloxy; gegebenenfalls durch $C_1$-$C_4$-Alkyl, $C_1$-$C_4$-Alkoxy oder Halogen substituiertes Benzyloxy oder Phenylethyloxy,

$Q_1$ Cyano; $C_1$-$C_{12}$-Alkoxycarbonyl; Benzyloxycarbonyl; gegebenenfalls durch Chlor oder $C_1$-$C_4$-Alkoxy substituiertes N,N-Di-$C_1$-$C_4$-Alkylcarbamoyl oder $C_1$-$C_4$-Alkylcarbonyl; gegebenenfalls durch Chlor oder $C_1$-$C_4$-Alkoxy substituiertes $C_1$-$C_{12}$-Alkoxysulfonyl, $C_1$-$C_{12}$-Alkylsulfonyl oder gegebenenfalls durch Chlor, $C_1$-$C_4$-Alkyl oder $C_1$-$C_4$-Alkoxy substituiertes Benzylsulfonyl,

$R_1$ $C_1$-$C_{12}$-Alkyl oder Benzyl,

$R_2$ und $R_4$ unabhängig voneinander Wasserstoff, Chlor, $C_1$-$C_{12}$-Alkyl, $C_1$-$C_{12}$-Alkoxy, Phenoxy, Benzyloxy oder einen Rest der Formel

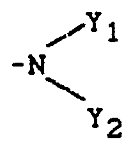

$R_3$ und $R_5$ unabhängig voneinander Wasserstoff; Chlor; $C_1$-$C_{12}$-Alkyl; $C_2$-$C_{12}$-Alkenyl; $C_1$-$C_{12}$-Alkoxy; $C_2$-$C_{12}$-Alkenyloxy; gegebenenfalls durch Chlor, $C_1$-$C_{12}$-Alkyl oder $C_1$-$C_{12}$-Alkoxy substituiertes Phenyl, Benzyl, Phenoxy oder Benzyloxy; gegebenenfalls durch $C_1$-$C_{12}$-Alkyl substituiertes Cyclohexyloxy oder Cyclopentyloxy; $C_1$-$C_{12}$-Alkylmercapto oder einen Rest der Formel

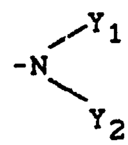

$Y_1$ und $Y_2$ unabhängig voneinander gegebenenfalls durch Chlor, Cyano, $C_1$-$C_4$-Alkoxycarbonyl oder $C_1$-$C_4$-Alkoxy substituiertes $C_1$-$C_{12}$-Alkyl; Cyclohexyl, Phenyl oder Benzyl, die durch Chlor, $C_1$-$C_{12}$-Alkyl oder $C_1$-$C_{12}$-Alkoxy substituiert sein können, oder

$R_2$ und $R_3$ Glieder, die zusammen mit Ring A' und/oder

$R_4$ und $R_5$ Glieder, die zusammen mit Ring B' zur Vervollständigung eines Ringsystems der folgenden Formeln

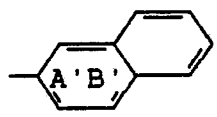 , 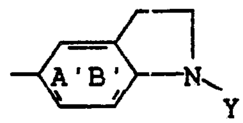 , 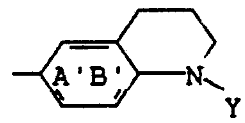 ,

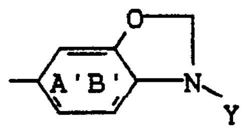 , 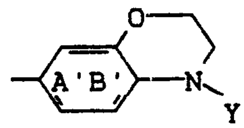 , 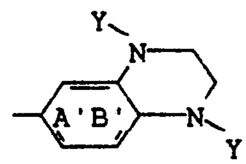 ,

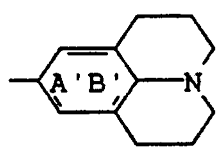 , 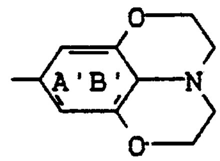 , 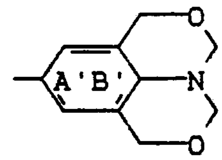 ,

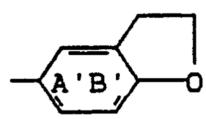 , 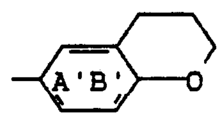 , 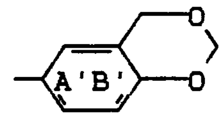 ,

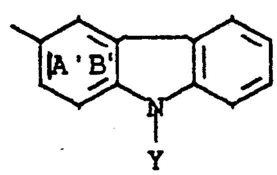 , 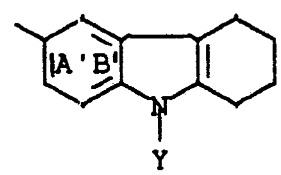 ,

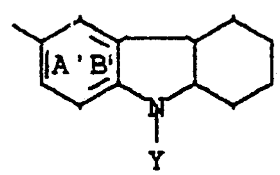 , 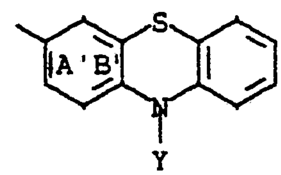 ,

erforderlich sind, bedeuten, worin

Y für $C_1$-$C_{12}$-Alkyl, das durch Chlor, Cyano, $C_1$-$C_4$-Alkoxycarbonyl oder $C_1$-$C_4$-Alkoxy substituiert sein kann; Cyclohexyl, Phenyl oder Benzyl, die durch Chlor, $C_1$-$C_{12}$-Alkyl oder $C_1$-$C_{12}$-Alkoxy substituiert sein können, steht, und

der gesättigte Ringteil bis zu 4 Reste aus der Gruppe Chlor, $C_1$-bis $C_4$-Alkyl, $C_1$-bis $C_4$-Alkoxy oder Phenyl tragen kann,

oder

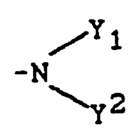

einen gegebenenfalls durch Chlor, $C_1$-bis $C_4$-Alkyl oder Phenyl substituierten Pyrrolo-, Pyrrolidino-, Piperidino-, Pipecolino-, Morpholino-, Pyrazolo-oder Pyrazolinrest bedeutet.

Beispiele für im gesättigten Ring substituierte Reste sind:

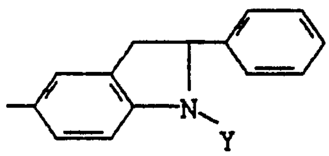 , 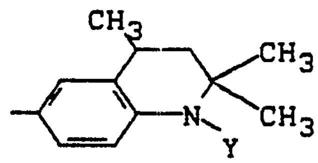 , 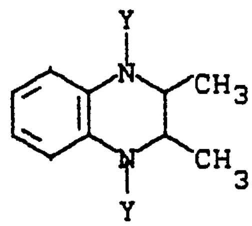 ,

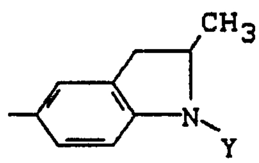 , 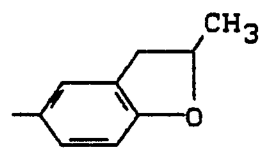 ,

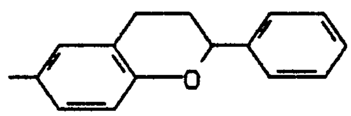 oder 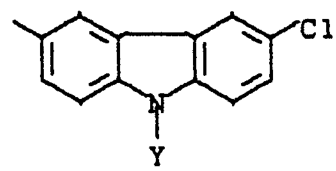

Besonders bevorzugt werden die Farbbildner der Formel

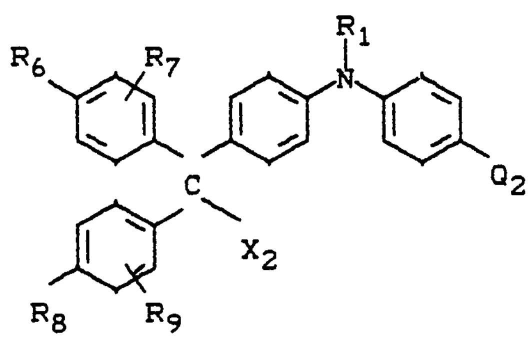 (III),

worin

$X_2$ Hydroxy oder $C_1$-$C_{12}$-Alkoxy,

$Q_2$ Cyano, $C_1$-$C_{12}$-Alkoxycarbonyl oder $C_1$-$C_4$-Alkylcarbonyl,

$R_6$ Wasserstoff, Chlor, $C_1$-$C_{12}$-Alkyl, $C_1$-$C_{12}$-Alkoxy, Benzyloxy oder einen Rest der Formel

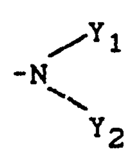

$R_8$ $C_1$-$C_{12}$-Alkoxy, Benzyloxy oder einen Rest der Formel

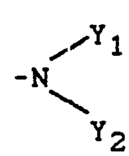

$R_7$ und $R_9$ unabhängig voneinander Wasserstoff, Chlor, $C_1$-$C_{12}$-Alkyl, $C_1$-$C_{12}$-Alkoxy, $C_1$-$C_4$-Alkylamino oder Di-$C_1$-$C_{12}$-Alkylamino oder

$R_6$ und $R_8$ Glieder, die zusammen mit dem Benzolring, an den sie gebunden sind, zur Vervollständigung eines Ringsystems der Formeln

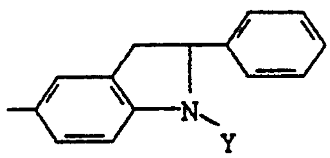 , 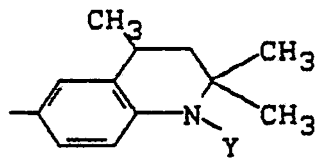 ,

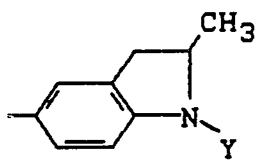 , 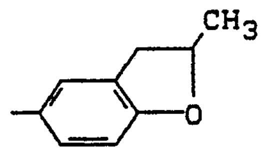 , 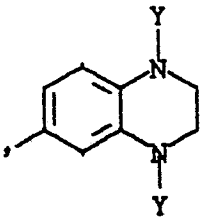 ,

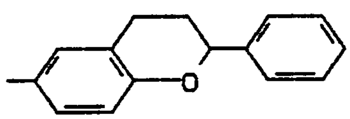 oder 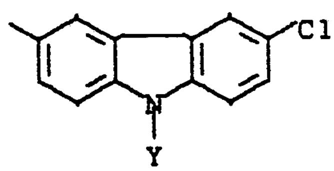

erforderlich sind, bedeuten und
$R^1$, Y, $Y_1$ und $Y_2$ die vorgenannte Bedeutung haben.

Die Farbbildner der Formel I können nach an sich bekannten Verfahren hergestellt werden. Ein Verfahren besteht z.B. darin, daß man Farbstoffsalze der Formel

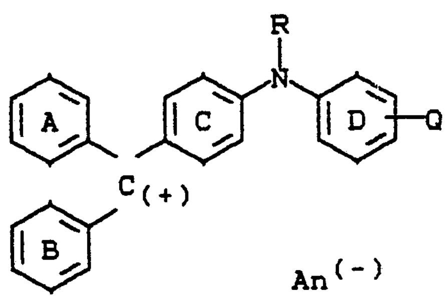 (IV),

mit Basen der Formel

MeX (V),

worin
R, X, Q und die Ringe A, B, C und D die obengenannte Bedeutung haben, und
Me ein Alkali-oder Erdalkalimetall, insbesondere Natrium oder Kalium und
$An^{(-)}$ ein Anion aus der Reihe Chlorid, Bromid, Acetat, Sulfat, Phosphat oder

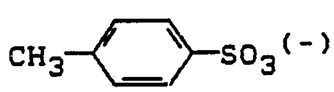

bedeuten,
in einem für die Reaktion geeigneten organischen Lösungsmittel umsetzt. Anschließend wird eventuell nach

Entfernen des Lösungsmittels auf Wasser ausgetragen und die Verbindungen der Formel I isoliert.

Geeignete Reaktionsmedium sind polare Lösungsmittel wie Dimethylformamid, Dimethylsulfoxid, Hexamethyl-phosphorsäure-triamid und Alkanole. Bevorzugt sind Dimethylformamid und $C_1$-$C_{18}$-Alkanole.

Geeignete Reaktionstemperaturen liegen zwischen 20 und 120°C, bevorzugt sind 30 bis 80°C.

Ein besonders vorteilhaftes Herstellungsverfahren besteht darin, daß man in einer Eintopfreaktion Ketone der Formel

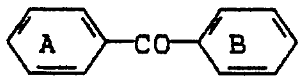

VI

mit Aminen der Formel

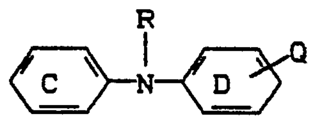

VII

oder Ketone der Formel

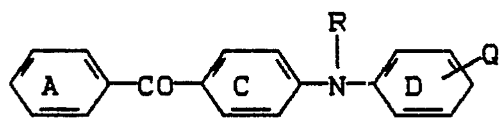

VIII

mit Verbindungen der Formel

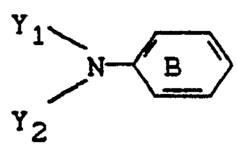

IX

in Gegenwart eines Kondensationsmittels, das ein Anion $An^{(-)}$ liefert zu den Farbsalzen der Formel (IV) kondensiert und diese ohne Zwischenisolierung mit den Basen (V) wie vorstehend beschrieben umsetzt.

Als Kondensationsmittel werden dabei vorzugsweise Phosphoroxychlorid und/oder Diphosphorpentoxid eingesetzt.

Die erhaltenen Farbbildner der Formel I können ohne weitere Reinigung in den Handel gebracht und in druck empfindlichen Aufzeichnungsmaterialien, insbesondere in mikroverkapselten Materialien eingesetzt werden.

Die erfindungsgemäßen Farbbildner der Formel I sind normalerweise farblos oder höchstens schwach gefärbt und zeigen eine sehr hohe Löslichkeit in den für die Verkapselung üblichen (Chlor)-Kohlenwasserstoffen.

Sie ergeben bei Kontakt mit einem sauren Entwickler, d.h. einem Elektronenakzeptor, intensive blaue, grünblaue, grüne, violette oder rote Farbtöne, die ausgezeichnet sublimations-und lichtecht sind. Durch Mischungen untereinander lassen sich marineblaue, graue oder schwarze Färbungen erzielen.

Sie sind auch wertvoll im Gemisch mit einem oder mehreren anderen bekannten Farbbildner, z.B. 3,3-Bis-(aminophenyl)-phthaliden, 3,3-Bis-(indolyl)-phthaliden, 3-Amino-fluoranen, 2,6-Diamino-fluoranen, Leukoauraminen, Spiropyranen, Spirodipyranen, Chromenoindolen, Phenoxazinen, Phenothiazinen, Carbazolylmethanen oder anderen Triarylmethanleukofarbstoffen, um grüne, violette, blaue, marineblaue, graue oder - schwarze Färbungen zu ergeben.

Sie zeigen sowohl auf phenolischen Unterlagen als auch auf Salicylat und aktivierten Tonen eine hohe Farbintensität, eine herausragende Lichtechtheit und ausgezeichnete Alterungs-und CB-Stabilität. Sie eignen sich für druckempfindliches Aufzeichnungsmaterial, das sowohl als Kopier-als auch Registriermaterial sein kann. Ihre Entwicklungsgeschwindigkeit ist in Abhängigkeit von den Substituenten unterschiedlich. Im allgemeinen zeichnen sie sich jedoch durch eine hohe Entwicklungsgeschwindigkeit aus bei gleichzeitig

reduzierter Empfindlichkeit der Aufzeichnungsmaterialien gegenüber unbeabsichtigter vorzeitiger Entwicklung.

Durch Substitution mit langkettigen Alkylresten wird die Löslichkeit der Farbbildner so erhöht, daß sich hochkonzentrierte Lösungen in der zur Mikroverkapselung geeigneten Lösungsmittel herstellen lassen.

Druckempfindliche Aufzeichnungsmaterialien sind beispielsweise aus den US-Patentschriften 2 800 457 und 2 800 458 bekannt.

Beispiel 1

13,5 g (0.05 Mol) 4,4'-Diethoxybenzophenon und 10,4 g (0,05 Mol) N-Methyl-N-phenyl-4-cyano-phenylamin werden in 38,2 g (0,25 Mol) Phosphoroxychlorid suspendiert und bei Raumtemperatur mit 14,1 g (0,1 Mol) Phosphorpentoxid versetzt. Es wird 20 Stunden bei 40°C verrührt. Man trägt auf 500 ml Eiswasser aus und rührt ca. 10-15 Stunden bei Raumtemperatur bis der Farbstoff sich kristallin abscheidet. Absaugen, Waschen mit Wasser und Trocknen im Vakuum bei 40°C liefert 24,1 g (97 % der Theorie) dunkelrotviolette Kristalle vom Schmp.: 69-75°C.

Eine Lösung von 19,9 g (0,04 Mol) dieses Farbstoffes in 200 ml Ethanol wird bei Raumtemperatur langsam zu 120 ml (0,12 Mol) einer vorgelegten 1 molaren Natriumethylat-Lösung getropft. Man rührt 20 Stunden bei Raumtemperatur, filtriert vom ausgefallenen Salz ab und entfernt das Lösungsmittel. Der verbleibende, ölige Rückstand wird in 500 ml Wasser eingerührt, auf ca. 10 bis 15°C abgekühlt und abfiltriert. Nach dem Trocknen im Vakuum bei Raumtemperatur erhält man 18,5 g (91 % der Theorie) eines fast farblosen Kristallpulvers vom Schmp.: 48-55°C und der Formel

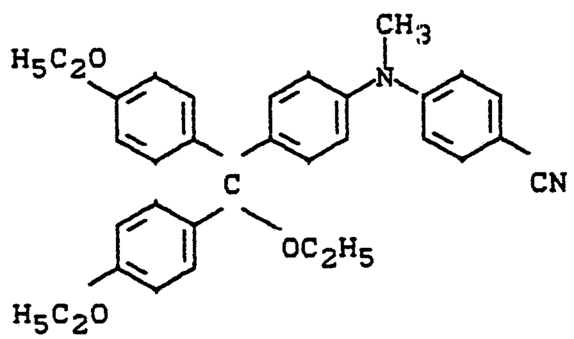

(1).

Eine Lösung in Eisessig wird dunkelviolett mit $\lambda_{max}$ = 554 nm und $\lambda_2$ = 442 nm
IR-Spektrum(KBr-Pressling): 2210 $cm^{-1}$ -CN;
$^{1}H$-NMR-Spektrum($CDCl_3$): $\delta$ = 1,22 ppm(T,3H) $\delta$ = 1,3 ppm(T,6H), $\delta$ = 3,1 ppm(Q,2H), $\delta$ = 3,3 ppm-(S,3H) $\delta$ = 4 ppm(Q,4H)
Mass. Spektrum: $C_{33}H_{34}N_2O_3$(506,7), m/e 506(12 %) $M^+$, m/e 461(100%) M±$OC_2H_5$
Auf Säureton wird ein kräftiger rötlich grau-schwarzer Farbton entwickelt.

Beispiel 2

Verfährt man wie im obigen Beispiel beschrieben, verwendet statt 4,4'-Diethoxybenzophenon 14,3 g (0,05 Mol) 4-Methoxy-4'-methoxyethoxybenzophenon und arbeitet statt mit Natriumethylat/Ethanol mit Natrium-iso-propylat/Isopropanol so erhält man 19,5 g (72 % der Theorie) beige Kristalle der Formel

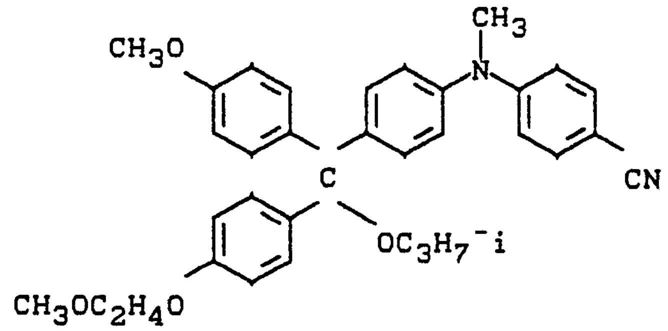

(2),

die auf Säureton und Phenolharz einen grau-schwarzen Farbton zeigen. Absorption in Eisessig $\lambda_{max}$ = 556 nm und $\lambda_2$ = 440 nm.

Beispiel 3

Verwendet man, wie im Beispiel 1 beschrieben, bei der Herstellung des Farbstoffes statt 13,5 g 4,4'-Diethoxybenzophenon 13,6 g (0,05 Mol) 2,4,4'-Trimethoxybenzophenon, so liefert die Umsetzung 23 g (92,4 % der Theorie) des entsprechenden Farbstoffes in Form von rotbraunen, metallisch-glänzenden Kristallen.

10 g (0,02 Mol) dieses Farbstoffes werden in 100 ml Dimethylformamid gelöst und bei Raumtemperatur langsam mit 10 g 20 %iger Natronlauge versetzt. Nach 2 Stunden Rühren wird die Reaktionsmischung auf 1 l Eiswasser ausgetragen. Nach Zugabe von 50 g Natriumchlorid wird der farblose kristalline Niederschlag abgesaugt. Trocknen im Vakuum bei 40°C liefert 8,8 g (91,7 % der Theorie) farblose Kristalle vom Schmp.: 68-80°C und der Formel

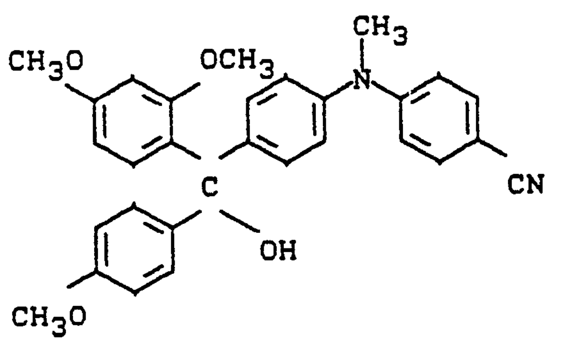

(3),

die aus Isopropanol umgelöst wrden können. Schmp.: 78-81°C. In Eisessig wird ein dunkel-blauvioletter Farbton entwickelt mit $\lambda_{max}$ = 566 nm und $\lambda_2$ = 476 nm.

Das IR-Spektrum zeigt bei 2205 $cm^{-1}$ die charakteristische CN-Bande und das $^1$H-NMR-Spektrum folgende Signale: $\delta$ = 5,1 ppm(S,1H); $\delta$ = 3,33(S,3H) $\delta$ = 3,67 ppm(S,3H), $\delta$ = 3,79(S,3H) und $\delta$ = 3,81-(S,3H). Mass-Spektrum: $C_{30}H_{28}N_2O_4$(480,6) m/e 480(27 %) $M^+$, m/e 463(15 %) $M^+$ -OH.

Auf Säureton oder Bisphenol A erhält man einen blauvioletten Farbton mit herausragenden CF-und CB-Stabilitäten.

Beispiel 4

Eine Lösung von 8,32 g (0,04 Mol) N-Methyl-N-phenyl-4-cyanophenylamin in 30,6 g Phosphoroxychlorid wird bei Raumtemperatur nacheinander mit 11,4 g (0,04 Mol) 4-Ethoxy-4'-ethylmercapto-benzophenon und 11,4 g Phosphorpentoxid versetzt. Die Reaktionsmischung wird auf 40°C erwärmt und 16 Stunden bei dieser Temperatur verrührt. Danach wird die auf 20°C abgekühlte Schmelze unter Kühlung in 150 ml Dimethylformamid gelöst. Unter starkem Rühren werden 24 g (0,12 Mol) 20 %ige Natronlauge so

zugetropft, daß sich eine Temperatur von 40°C einstellt. Man rührt noch 1 Stunde bei 40°C nach, filtriert vom Salz ab und gibt die Lösung auf 500 ml Eiswasser. Nach Zugabe von 25 g Natriumchlorid wird der farblose kristalline Niederschlag abgesaugt, mit 1 %iger Natronlauge und Wasser gewaschen. Nach Trocknen im Vakuum erhält man 14,3 g (72,4 % der Theorie) des Farbbildners der Formel

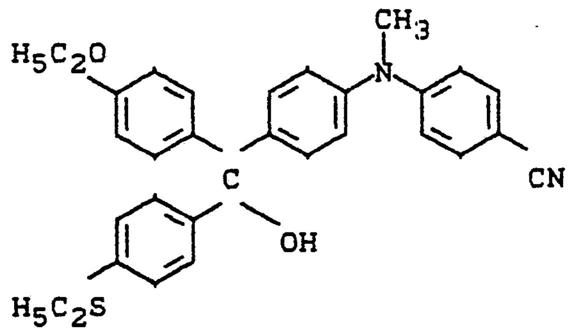

(4),

mit einem Schmelzbereich: 59-70°C. Eine Lösung in Eisessig ist blau mit $\lambda_{max}$ = 576 nm und $\lambda_2$ = 480 nm. Das IR-Spektrum zeigt die charakteristische CN-Bande bei 2221 $cm^{-1}$ und das Mass-Spektrum liefert m/e 494(48 %) $M^+$ und m/e 478(16 %) $M^+$-OH. ($C_{31}H_{30}N_2SO_2$(404,7).

Auf Säureton erhält man ein sehr stabiles Blau.

Beispiel 5

Analog Beispiel 1-4 werden auch die folgenden Farbbildner hergestellt.

Tabelle 1

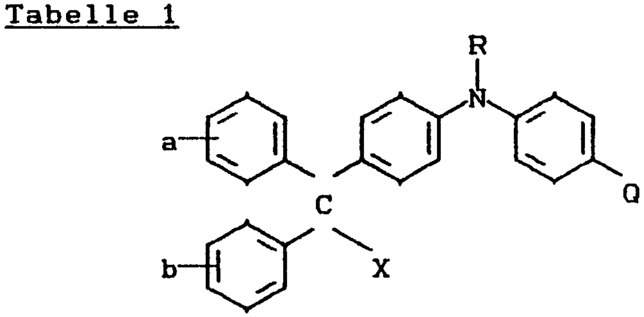

| Formel Nr. | a | b | R | Q | X | Farbton auf Säureton oder Bisphenol A |
|---|---|---|---|---|---|---|
| 5 | 4-$OCH_3$ | 4-$OCH_3$ | $CH_3$ | CN | $OC_4H_9$-n | schwarz-grau |
| 6 | 4-$OCH_3$ | 4-$OCH_3$ | $CH_2$-$C_6H_5$ | CN | $OC_2H_5$ | schwarz-grau |
| 7 | 4-$OCH_3$ | 3-$OCH_3$<br>4-$OCH_3$ | $CH_3$ | CN | $OCH_3$ | grau-blau |
| 8 | 3-$OCH_3$ | 3-$OCH_3$<br>4-$OCH_3$ | $C_2H_5$ | CN | $OC_2H_5$ | blau |
| 9 | 4-$OCH_3$ | 3-$CH_3$<br>4-$SCH_3$ | $CH_3$ | CN | OH | dunkelrotviolett |
| 10 | 4-$OCH_3$ | 2-$OCH_3$<br>3-$OCH_3$<br>4-$OCH_3$ | $CH_3$ | CN | $OC_3H_7$-i | violett |
| 11 | 4-$OCH_3$ | 3-$C_3H_7$-i<br>4-$OCH_3$<br>3-$C_3H_7$-i | $CH_3$ | CN | $OC_3H_7$-i | blau-schwarz |
| 12 | 4-$OC_2H_5$ | 4-$OC_2H_5$ | $CH_3$ | CO-$CH_3$ | $OC_2H_5$ | grau-schwarz |
| 13 | 2-$CH_3$<br>4-$OCH_3$ | 3-$OCH_3$ | $CH_2$-$C_6H_5$ | $COOCH_3$ | $OCH_3$ | rot |
| 14 | 2-Cl<br>4-$OC_3H_7$ | 3-$OCH_3$<br>4-$OCH_3$ | $CH_3$ | $COOC_2H_5$ | $OC_6H_{13}$ | dunkelblauviolett |
| 15 | 4-$C_{12}H_{25}$ | 2-$OCH_3$<br>4-$OCH_3$ | $C_4H_9$ | CN | $OC_4H_9$ | dunkelrot |

Beispiel 6

13,6 g (0,04 Mol) 2,4'-Diethoxy-4-diethylamino-benzophenon und 8,3 g (0,04 Mol) N-Methyl-N-phenyl-4-cyanophenylamin werden in 30,6 g (0,2 Mol) Phosphoroxychlorid suspendiert und langsam mit 11,3 g (0,08 Mol) Phosphorpentoxid versetzt. Die Reaktionsmischung rührt 20 Stunden bei 40°C. Danach wird die Schmelze auf 600 ml Eiswasser ausgetragen, 10 Stunden verrührt und der dann kristalline Niederschlag abgesaugt. Nach dem Trocknen im Vakuum bei 40°C erhält man 18,8 g (82,8 % der Theorie) bräunliches Kristallpulver.

17 g (0,03 Mol) dieses Farbstoffes werden in 70 ml Dimethylformamid gelöst und abfiltriert. Zur Lösung werden innerhalb 1 Stunde 15 g 20 %ige Natronlauge zugetropft. Man rührt 2 Stunden bei 40°C, filtriert ab und trägt die Lösung in 500 ml Eiswasser ein. Nach Zugabe von 40 g Natriumchlorid wird kurz verrührt und abgesaugt. Der Filterkuchen wird dann in 50 ml Methanol suspendiert und erneut abgesaugt. Nach Trocknen im Vakuum erhält man 9,5 g (57,6 % der Theorie) hellgraues Kristallpulver mit einem Schmp.: 147-54°C und der Formel

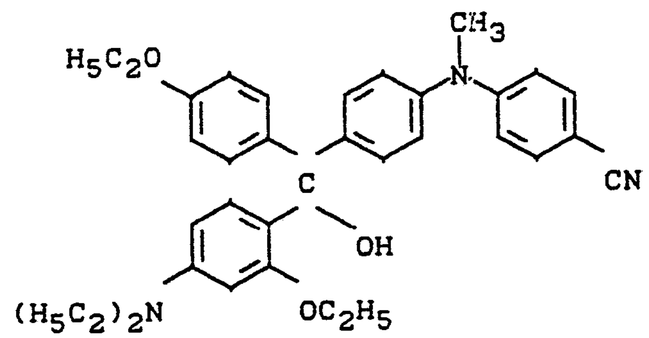

(16),

das in Eisessig gelöst eine blaue Farbe mit $\lambda_{max}$ = 604 nm und $\lambda_2$ = 477 nm zeigt.

Auf Säureton erhält man eine farbstarke dunkelblaue Färbung mit ausgezeichneter Lichtechtheit und Alterungsstabilität.

Beispiel 7

14,4 g (0,05 Mol) 4-(N-Methyl-N-phenylamino)-benzophenon und 10,4 g (0,05 Mol) N-Methyl-N-phenyl-4-cyanophenylamin werden in 38,2 g Phosphoroxychlorid suspendiert und bei Raumtemperatur mit 14,1 g Phosphorpentoxid versetzt. Die Mischung wird 20 Stunden bei 40°C verrührt und unter Außenkühlung in 250 ml Methanol gelöst, so daß die Temperatur 50°C nicht übersteigt. Unter gutem Rühren werden 24 g (0,12 Mol) 20 %ige Natronlauge zugetropft, wobei die Temperatur zwischen 40-50°C bleibt. Die Eintropfzeit beträgt 1 Stunde. Die Mischung wird 5 Stunden bei 40-50°C verrührt und mit 250 ml Wasser versetzt. Es wird auf 10-15°C abgekühlt und der fast farblose Niederschlag abgesaugt. Nach Waschen mit Methanol/Wasser und Trocknen erhält man 18,3 g (73,8 % der Theorie) fast farbloser Kristalle vom Schmelzbereich: 55-61°C und der Formel, die aus einem Gemisch von Carbinolbasenmethylether (ca. 80 %) und der Carbinolbase (ca. 20 %) der Formel

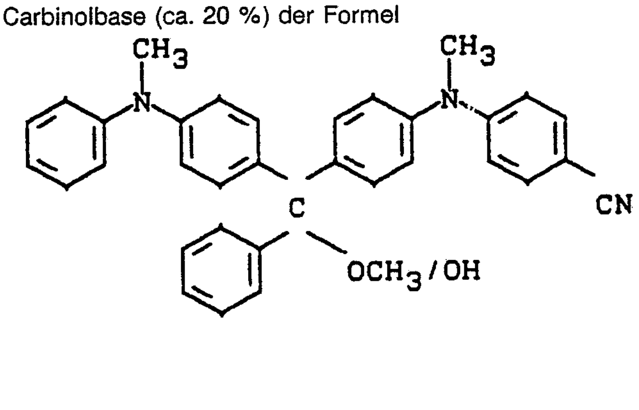

(17),

bestehen. Absorption in Eisessig: $\lambda_{max}$ = 626 nm; $\lambda_2$ = 437 nm.

Auf Säureton und Phenolharz entwickelt der Farbbildner einen kräftigen, grünen Farbton mit guten Echtheiten.

Beispiel 8

Auf gleiche Weise, wie im Beispiel 6 und 7 beschrieben, erhält man auch die folgenden Farbbildner der Tabelle 2.

Tabelle 2

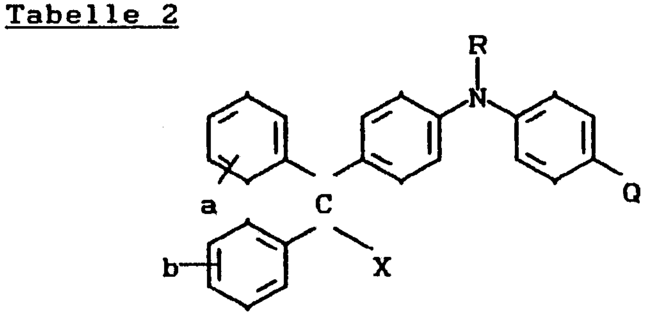

| Formel Nr. | a | b | R | Q | X | Farbton auf Säureton oder Bisphenol A |
|---|---|---|---|---|---|---|
| 18 | 4-$OCH_3$ | 4-$N(CH_3)_2$ | $C_4H_9$ | CN | $OCH_3$ | grau-blau |
| 19 | 4-$OC_2H_5$ | 2-$CH_3$<br>4-$N(C_2H_5)_2$ | $CH_3$ | CN | $OCH_3$ | blau |
| 20 | 2-$OCH_3$ | 4-N($C_2H_5$)($CH_2$-$C_6H_5$) | $CH_3$ | CN | $OC_6H_{13}$ | grün |
| 21 | 4-$OCH_3$ | 2-$CH_3$<br>4-N($C_2H_5$)($C_2H_4$-Cl) | $CH_3$ | CN | OH | blau |
| 22 | 2-Cl<br>4-Cl | 4-N($C_2H_5$)($C_2H_4$-CN) | $C_2H_5$ | CN | $OC_2H_5$ | grün |

<u>Tabelle 2</u> (Fortsetzung)

| Formel Nr. | a | b | R | Q | X | Farbton auf Säureton oder Bisphenol A |
|---|---|---|---|---|---|---|
| 23 | 4-$OC_4H_9$ | 4-$N(C_4H_9)_2$ | $CH_3$ | $COOC_8H_{17}$ | $OH/OCH_3$ | grau-blau |
| 24 | 2-Cl | 4-$N(CH_3)_2$<br>2-$CH_3$<br>5-$OCH_3$ | $CH_2$-$C_6H_5$ | $COOC_8H_{17}$ | $OC_2H_5/OH$ | dunkelgrün |
| 25 | 4-$OCH_3$ | 2-$N(CH_3)_2$<br>4-$N(CH_3)_2$<br>5-$CH_3$ | $C_4H_9$ | CN | $OC_4H_9/OH$ | schwarzgrau |
| 26 | 4-$OC_2H_5$ | 4-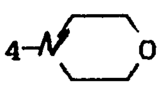 | $CH_3$ | CO-$C_4H_9$ | OH | blauviolett |
| 27 | 4-$N(C_2H_5)_2$ | 4-N($C_2H_5$)($CH_2$-$C_6H_5$)<br>2-$CH_3$ | $CH_3$ | CN | $OC_2H_5$ | dunkelblau |
| 28 | 3-Cl<br>4-$OCH_3$ | 4-N($C_6H_5$)($CH_3$) | $CH_3$ | $COOC_2H_5$ | $OC_2H_5/OH$ | dunkelgrün |
| 29 | 4-$OC_2H_5$ | 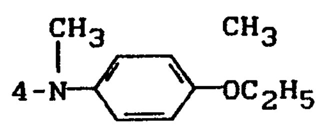 | $CH_3$ | CN | $OC_8H_{17}$ | graublau |

Tabelle 2 (Fortsetzung)

| Formel Nr. | a | b | R | Q | X | Farbton auf Säureton oder Bisphenol A |
|---|---|---|---|---|---|---|
| 30 | 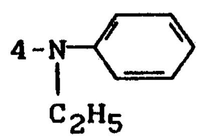 | 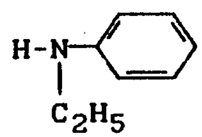 | $C_2H_5$ | $COOC_4H_9$ | $OC_2H_5$ | dunkelblau |
| 31 | 4-$OC_2H_5$ | 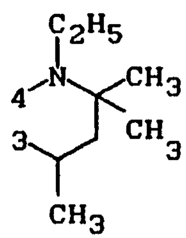 | $CH_3$ | CN | $OCH_3$ | schwarz-blau |
| 32 | 4-$OCH_3$ | 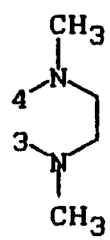 | $CH_3$ | CN | $OC_4H_9$ | grün-schwarz |

## Ansprüche

1. Farbbildner der allgemeinen Formel,

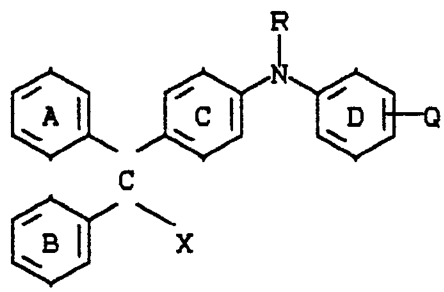

worin

X Hydroxy, Alkoxy, Alkenyloxy, Aralkoxy, Cycloalkoxy, Aryloxy, Acyloxy, Alkoxycarbonyloxy, Alkylamino, Dialkylamino, Acylamino, Aralkylamino oder Arylamino,

Q Cyano, Alkoxycarbonyl, Aryloxycarbonyl, Aralkoxycarbonyl, Carbamoyl, N-Alkylcarbamoyl, N,N-Dialkylcarbamoyl, N-Alkyl-N-aryl-carbamoyl, Acyl, Alkoxysulfonyl, Aralkoxysulfonyl, Sulfamoyl, N-Alkylsulfamoyl, N,N-Dialkylsulfamoyl, Alkylsulfonyl, Arylsulfonyl oder Aralkylsulfonyl und

R Alkyl, Alkenyl oder Aralkyl bedeuten,

den Ringen A, B, C und D weitere isocyclische oder heterocyclische Ringe anelliert werden können, und die cyclischen und acyclischen Reste sowie die Ringe A, B, C und D weitere in der Farbstoffchemie übliche, nichtionische Substituenten tragen können, oder deren Gemische.

2. Farbbildner gemäß Anspruch 1 der Formel,

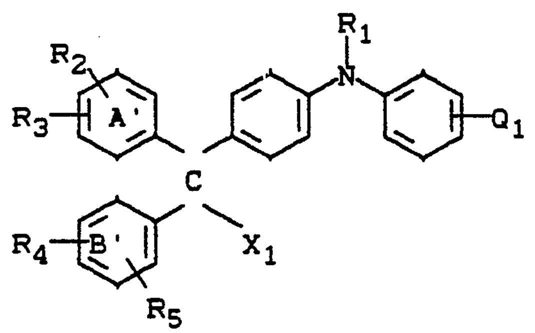

worin

$X_1$ Hydroxy; gegebenenfalls durch Chlor oder $C_1$-$C_4$-Alkoxy substituiertes $C_1$-$C_{12}$-Alkoxy; $C_2$-$C_{12}$-Alkenyloxy; gegebenenfalls durch $C_1$-$C_4$-Alkyl, $C_1$-$C_4$-Alkoxy oder Halogen substituiertes Benzyloxy oder Phenylethyloxy,

$Q_1$ Cyano; $C_1$-$C_{12}$-Alkoxycarbonyl; Benzyloxycarbonyl; gegebenenfalls durch Chlor oder $C_1$-$C_4$-Alkoxy substituiertes N,N-Di-$C_1$-$C_4$-Alkylcarbamoyl oder $C_1$-$C_4$-Alkylcarbonyl; gegebenenfalls durch Chlor oder $C_1$-$C_4$-Alkoxy substituiertes $C_1$-$C_{12}$-Alkoxysulfonyl, $C_1$-$C_{12}$-Alkylsulfonyl oder gegebenenfalls durch Chlor, $C_1$-$C_4$-Alkyl oder $C_1$-$C_4$-Alkoxy substituiertes Benzylsulfonyl,

$R_1$ $C_1$-$C_{12}$-Alkyl oder Benzyl,

$R_2$ und $R_4$ unabhängig voneinander Wasserstoff, Chlor, $C_1$-$C_{12}$-Alkyl, $C_1$-$C_{12}$-Alkoxy, Phenoxy, Benzyloxy oder einen Rest der Formel

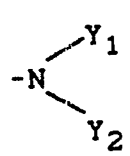

$R_3$ und $R_5$ unabhängig voneinander Wasserstoff; Chlor; $C_1$-$C_{12}$-Alkyl; $C_2$-$C_{12}$-Alkenyl; $C_1$-$C_{12}$-Alkoxy; $C_2$-$C_{12}$-Alkenyloxy; gegebenenfalls durch Chlor, $C_1$-$C_{12}$-Alkyl oder $C_1$-$C_{12}$-Alkoxy substituiertes Phenyl, Benzyl,

Phenoxy, oder Benzyloxy; gegebenenfalls durch $C_1$-$C_{12}$-Alkyl substituiertes Cyclohexyloxy oder Cyclopentyloxy; $C_1$-$C_{12}$-Alkylmercapto oder einen Rest der Formel

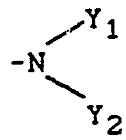

$Y_1$ und $Y_2$ unabhängig voneinander gegebenenfalls durch Chlor, Cyano, $C_1$-$C_4$-Alkoxycarbonyl oder $C_1$-$C_4$-Alkoxy substituiertes $C_1$-$C_{12}$-Alkyl; Cyclohexyl, Phenyl oder Benzyl, die durch Chlor, $C_1$-$C_{12}$-Alkyl oder $C_1$-$C_{12}$-Alkoxy substituiert sein können, oder

$R_2$ und $R_3$ Glieder, die zusammen mit Ring A' und/oder

$R_4$ und $R_5$ Glieder, die zusammen mit Ring B' zur Vervollständigung eines Ringsystems der folgenden Formeln

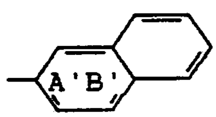 , 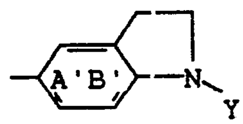 , 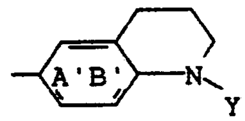 ,

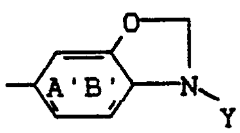 , 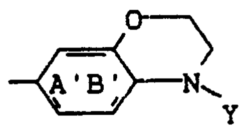 , 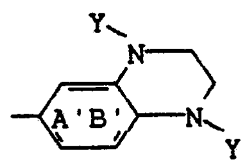 ,

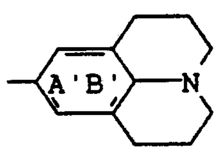 , 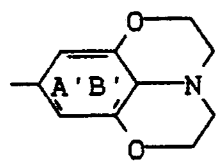 , 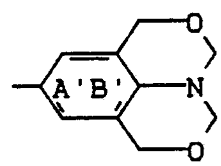 ,

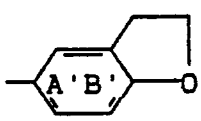 , 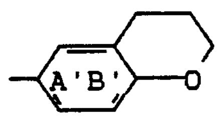 , 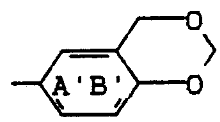 ,

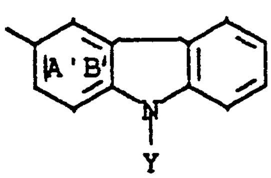 , 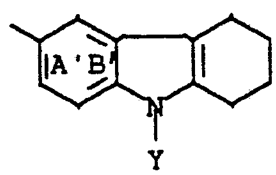 ,

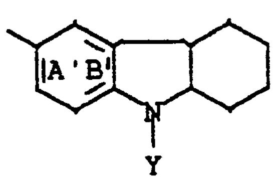 , 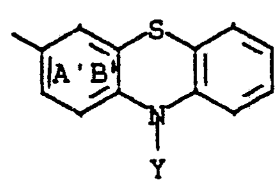 ,

erforderlich sind, bedeuten, worin

Y für $C_1$-$C_{12}$-Alkyl, das durch Chlor, Cyano, $C_1$-$C_4$-Alkoxycarbonyl oder $C_1$-$C_4$-Alkoxy substituiert sein

kann; Cyclohexyl, Phenyl oder Benzyl, die durch Chlor, $C_1$-$C_{12}$-Alkyl oder $C_1$-$C_{12}$-Alkoxy substituiert sein können, steht,
der gesättigte Ringteil bis zu 4 Reste aus der Gruppe Chlor, $C_1$-bis $C_4$-Alkyl, $C_1$-bis $C_4$-Alkoxy oder Phenyl tragen kann,
oder

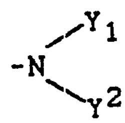

einen gegebenenfalls durch Chlor, $C_1$-bis $C_4$-Alkyl oder Phenyl substituierten Pyrrolo-, Pyrrolidino-, Piperidino-, Pipecolino-, Morpholino-, Pyrazolo-oder Pyrazolinrest bedeutet.

3. Farbbildner gemäß Anspruch 1 der Formel,

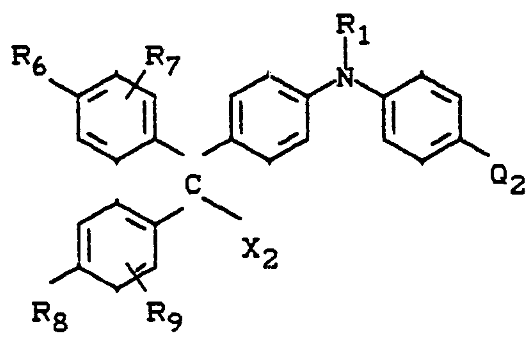

worin

$X_2$ Hydroxy oder $C_1$-$C_{12}$-Alkoxy,

$Q_2$ Cyano, $C_1$-$C_{12}$-Alkoxycarbonyl oder $C_1$-$C_4$-Alkylcarbonyl,

$R_6$ Wasserstoff, Chlor, $C_1$-$C_{12}$-Alkyl, $C_1$-$C_{12}$-Alkoxy, Benzyloxy oder einen Rest der Formel

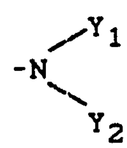

$R_8$ $C_1$-$C_{12}$-Alkoxy, Benzyloxy oder einen Rest der Formel

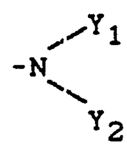

$R_7$ und $R_9$ unabhängig voneinander Wasserstoff, Chlor, $C_1$-$C_{12}$-Alkyl, $C_1$-$C_{12}$-Alkoxy, $C_1$-$C_4$-Alkylamino oder Di-$C_1$-$C_{12}$-Alkylamino oder

$R_6$ und $R_8$ Glieder, die zusammen mit dem Benzolring, an den sie gebunden sind, zur Vervollständigung eines Ringsystems der Formeln

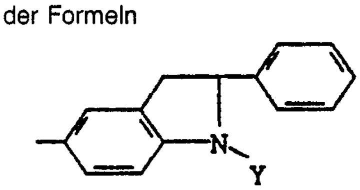 , 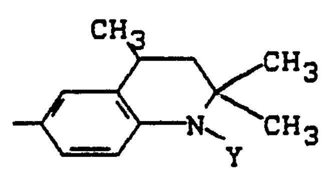 ,

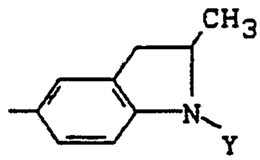 , 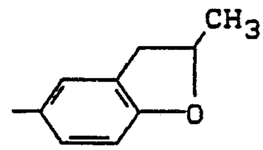 , 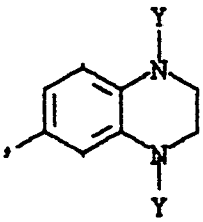 ,

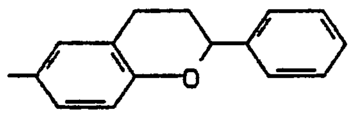 oder 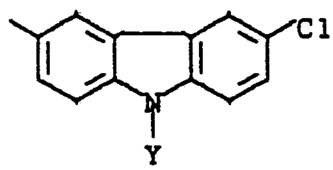

erforderlich sind, bedeuten und
$R^1$, Y, $Y_1$ und $Y_2$ die vorgenannte Bedeutung haben.

4. Verfahren zur Herstellung von Farbbildnern gemäß Anspruch 1, dadurch gekennzeichnet, daß man in einer Eintopfreaktion Ketone der Formel

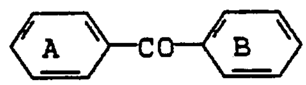

mit Aminen der Formel

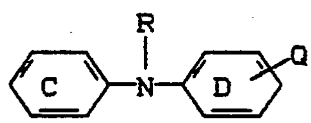

oder Ketone der Formel

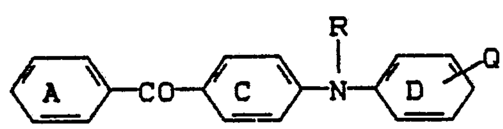

mit Verbindungen der Formel

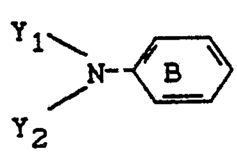

in Gegenwart eines Kondensationsmittels, das ein Anion $An^{(-)}$ liefert zu den Farbsalzen der Formel

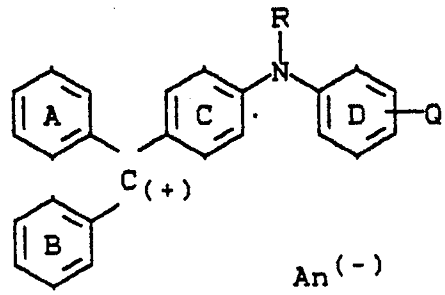

kondensiert und diese ohne Zwischenisolierung mit Basen der Formel

MeX,

worin R, X, Q und die Ringe A, B, C und D die in Anspruch 1 angegebene Bedeutung haben,
Me ein Alkali-oder Erdalkalimetallion und
$An^{(-)}$ ein Anion aus der Gruppe Chlorid, Bromid, Acetat, Sulfat, Phosphat oder

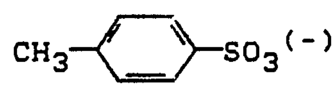

bedeuten, umsetzt.

5. Verwendung eines Farbbildners gemäß Anspruch 1 in druck-, wärme-und elektrosensitivem Aufzeichnungsmaterial.

6. Druck-, wärme-und elektrosensitives Aufzeichnungsmaterial, dadurch gekennzeichnet, daß es in seinem Reaktantensystem mindestens einen Farbbildner gemäß Anspruch 1 enthält.